# EUROPEAN PATENT SPECIFICATION

(11) **EP 3 455 821 B1**
(45) Date of publication and mention of the grant of the patent: **19.02.2025**
(21) Application number: 17824544.5
(22) Date of filing: 06.07.2017
(51) Int. Cl.: G06Q 30/0203, G16H 20/70, G16H 40/63, G16H 40/67, G16H 50/30, G16H 50/70, G16H 80/00, G06Q 10/04, G06Q 50/22

(54) **AUTOMATICALLY DETERMINING AND RESPONDING TO USER SATISFACTION**
AUTOMATISCHE BESTIMMUNG VON UND REAKTION AUF BENUTZERZUFRIEDENHEIT
DÉTERMINATION ET RÉPONSE AUTOMATIQUES À LA SATISFACTION DE L'UTILISATEUR

(30) Priority: 06.07.2016 US 201662359119 P; 27.02.2017 US 201715444231
(43) Date of publication of application: 20.03.2019
(73) Proprietor: Samsung Electronics Co., Ltd., Suwon-si, Gyeonggi-do 16677 (KR)
(72) Inventor: PHAN, Thomas, Mountain View, California 94043 (US)
(74) Representative: HGF
(86) International application number: PCT/KR2017/007200
(87) International publication number: WO 2018/008988

(56) References cited:
- WO-A1-2014/190201
- US-A1- 2008 177 836
- US-A1- 2010 332 259
- US-A1- 2013 144 638
- US-A1- 2014 214 443
- No further relevant documents disclosed

## Description

### Technical Field

One or more embodiments relate generally to activity and communication monitoring and analysis, and in particular, automatically determining and responding to user satisfaction.

### Background Art

A plan usually is a program outlining a set of actions/activities that an individual should perform/participate in to change his/her status. For example, a wellness plan is a plan that aims to improve or maintain an individual's physical wellness (e.g., a plan to achieve a desired fitness or health goal).

### Disclosure of Invention

### Technical Problem

There is a need for a system and a method to determine an individual's satisfaction with a plan in which he/she is involved, and to notify in a timely manner an administrator of the plan and/or another third party member to take timely reaction to ensure effectiveness of the plan.

### Brief Description of Drawings

FIG. 1A illustrates an example computing framework for implementing a satisfaction determination system, in one or more embodiments;
FIG. 1B illustrates another example computing framework for implementing a satisfaction determination system, in one or more embodiments;
FIG. 2A illustrates one or more example components of the satisfaction determination system utilized in a training phase, in one or more embodiments;
FIG. 2B illustrates one or more example components of the satisfaction determination system utilized in a determination phase, in one or more embodiments;
FIG. 3 illustrates an example of different types of data collected by the satisfaction determination system, in one or more embodiments;
FIG. 4A illustrates an example software user interface displayed on a smartphone, in one or more embodiments;
FIG. 4B illustrates another example software user interface displayed on a smartphone, in one or more embodiments;
FIG. 4C illustrates yet another example software user interface displayed on a smartwatch, in one or more embodiments;
FIG. 5 illustrates one or more examples processes performed by the satisfaction determination system during the training phase, in one or more embodiments;
FIG. 6 illustrates one or more examples processes performed by the satisfaction determination system during the determination phase, in one or more embodiments;
FIG. 7 illustrates an example alert engine in detail, in one or more embodiments;
FIG. 8 illustrates an example software user interface providing an alert, in one or more embodiments;
FIG. 9 illustrates an example personalized corrective recommendation engine in detail, in one or more embodiments;
FIG. 10 illustrates an example overall user satisfaction score with a wellness plan based on user satisfaction with specific, granular aspects of the wellness plan, in one or more embodiments;
FIG. 11A is a flowchart of an example process for determining user satisfaction, in one or more embodiments;
FIG. 11B is a flowchart of an example process for training a statistical machine learning model for use in determining user satisfaction, in one or more embodiments; and
FIG. 12 is a high-level block diagram showing an information processing system comprising a computer system useful for implementing the disclosed embodiments.

### Best Mode for Carrying out the Invention

One embodiment provides a method comprising receiving user data from one or more devices of a user, determining a satisfaction level of the user with a wellness plan based on a learned statistical model, and selectively sending a communication to a different user based on the satisfaction level.

Another embodiment provides a method comprising, in a training phase, collecting user data from a plurality of users. The user data collected comprises ground-truth user satisfaction data indicative of one or more satisfaction levels of one or more users with a wellness plan. The method further comprises extracting features from the user data, and training a statistical model based on the features extracted and the ground-truth satisfaction data. The statistical model maps a given set of extracted features to a corresponding satisfaction level.

These and other features, aspects and advantages of the one or more embodiments will become understood with reference to the following description, appended claims and accompanying figures.

### Mode for the Invention

The following description is made for the purpose of illustrating the general principles of one or more embodiments and is not meant to limit the inventive concepts claimed herein. Further, particular features described herein can be used in combination with other described features in each of the various possible combinations and permutations. Unless otherwise specifically defined herein, all terms are to be given their broadest possible interpretation including meanings implied from the specification as well as meanings understood by those skilled in the art and/or as defined in dictionaries, treatises, etc.

One or more embodiments relate generally to activity and communication monitoring and analysis, and in particular, automatically determining and responding to user satisfaction. One embodiment provides a method comprising receiving user data from one or more devices of a user, determining a satisfaction level of the user with a wellness plan based on a learned statistical model, and selectively sending a communication to a different user based on the satisfaction level.

Another embodiment provides a method comprising, in a training phase, collecting user data from a plurality of users. Some of the user data may be collected in a real-time manner. The user data collected comprises ground-truth user satisfaction data indicative of one or more satisfaction levels of one or more users with a wellness plan. The method further comprises extracting features from the user data, and training a statistical model based on the features extracted and the ground-truth satisfaction data. The statistical model maps a given set of extracted features to a corresponding satisfaction level.

For expository purposes, the term "device" as used in this specification generally refers to a mobile, wearable, or Internet-of-Things (IoT) device. Examples of devices include, but are not limited to, smart body patches, smartphones, smart watches, smart vehicles, smart houses, environmental IoT home sensors, etc.

For expository purposes, the term "physical wellness" as used in this specification generally encompass terms and scenarios that pertain to a physical state of healthy living, including mental wellness. Physical wellness is an important aspect of an individual's daily life. Examples of different application scenarios/contexts in which an individual is involved in physical wellness may include, but are not limited to, the following: fitness involving a fitness enthusiast who wants to exercise more to stay in shape or lose weight, medical rehabilitation involving a medical patient discharged from a hospital who follows a physical exercise plan prescribed by a doctor to recover from surgery or prevent chronic illnesses (e.g., diabetes, hypertension, etc.), elderly/ disabled care involving an elderly/disabled individual who wants to maintain or recover his/her level of physical activity, athletics involving an amateur/professional athlete who wants to increase strength or stamina, etc.

For expository purposes, the term "wellness plan" as used in this specification generally refers to a plan/program outlining a set of actions/activities that an individual should take/participate in to improve and/or maintain his/her physical wellness (e.g., a desired fitness or health goal). For expository purposes, the term "wellness manager" as used in this specification generally refers a person or entity that monitors/supervises an individual's progress with a wellness plan (i.e., an administrator of the plan). A wellness manager may define/design a wellness plan for an individual. Examples of different types of wellness managers include, but are not limited to, the following: doctors, physicians, nurses, physical therapists, trainers, family members, coaches, etc. A wellness plan may comprise multiple aspects, such as a medication aspect (i.e., medication prescribed), an exercise aspect (i.e., exercise recommended), a hardware and software aspect (i.e., devices and software applications utilized), and a human-interaction aspect with a wellness manager.

An individual may participate in a wellness plan defined by a wellness manager who supervises the progress of the individual. The wellness plan may include instructions that the individual should follow to achieve a desired goal. For example, in the context of light fitness or medical rehabilitation, an individual may follow a wellness plan defined by a physician or a physical therapist; the wellness plan may identify intensity and frequency of exercises that the individual should follow (e.g., running and reaching a 90 beats/minute heartrate four days a week). This wellness plan may derive from the user needing preventive care for chronic conditions such as obesity, hypertension, pre-hypertension, diabetes, and pre-diabetes, where the general prescription is increased daily exercise. As another example, a user who is recently discharged from cardiac surgery may require rehabilitation where a certain level of exercise exertion is needed in order to prevent readmission for further treatment. In the context of elderly care, a wellness plan defined by a doctor may require an elderly individual to take required medication on time and go out walking at least 5,000 steps daily. In the context of athletics, a wellness plan defined by a coach or trainer may identify that an amateur/professional athlete should partake in anaerobic weight-lifting for both upper and lower body three times a week.

Remote user monitoring is an increasingly valuable method for improving physical wellness of users, lowering cost of individualized care, and increasing efficacy of treatment. A wellness manager may assign one or more mobile/wearable devices (e.g. a smartphone, a smartwatch, etc.) or Internet-of-Things (IoT) devices (e.g., an environmental IoT sensor, etc.) to a user as part of a complete wellness plan to improve the user's health. Each assigned device collects sensor data from one or more sensors that capture metrics associated with various attributes of the user's daily physical activity, such as how many steps the user has walked, duration of exercise sessions the user has participated in, etc. The collected sensor data may be sent to an online service for review by the wellness manager to determine whether the user is adhering to the wellness plan and making progress toward a desired health or fitness goal.

A significant issue that may arise with respect to wellness plans is when an user becomes dissatisfied with his/her wellness plan, thereby causing a decline in progress and eventual failure to meet a goal of the wellness plan. The dissatisfaction may stem from many factors such as, but not limited to, novelty and perceived importance of the wellness plan may diminish after a period of time (e.g., several weeks), user frustration with pain or lack of health progress, user frustration with a mobile application or an assigned mobile device, the user may not have time to become fully engaged during a holiday season, etc. If the user becomes completely disengaged and fails to complete the wellness plan, the user's health may degrade, possibly resulting in a recurrence of a medical condition and a subsequent re-admission to a care facility (e.g., a hospital) for further treatment.

Conventionally, a wellness manager utilizes remote user monitoring to review raw sensor data capturing a user's physical activity (e.g., reviewing raw data trend lines of the user's step count over time) and determines whether the user is making progress towards a desired goal of a wellness plan (e.g., a health/fitness goal). This conventional approach has many drawbacks. For example, it may not be possible to determine user satisfaction with a wellness plan based on raw sensor data alone. Therefore, a wellness manager does not have any information about user satisfaction with a wellness plan, and may have to make a subjective guess for new actions that can improve user satisfaction. Even though a user may meet/achieve certain thresholds defined in a wellness plan (e.g., a specific number of steps to walk per day), the user may be dissatisfied with and may eventually drop out of the wellness plan at any time. There may be different reasons for the user dissatisfaction such as, but not limited to, the wellness plan is inconvenient to the user, the user dislikes the wellness manager, the user experiences too much pain, etc. Conversely, even though a user may not meet/achieve certain thresholds defined in a wellness plan, the user may be satisfied and wants to continue with the wellness plan. There may be different reasons for the user not meeting/achieving certain thresholds such as, but not limited to, user travel (e.g., on vacation or a business trip), bad weather, etc. An existing solution is to periodically survey/ask a user whether he/she feels satisfied with a wellness plan. This solution, however, may become tedious for both the user and the wellness manager over time as the wellness manager must frequently prompt the user to record his/her satisfaction, leading to engagement fatigue and an artificially low user satisfaction.

Another example drawback of the conventional approach is that it is not scalable. A wellness manager may manage/supervise a large number of users (e.g., over 1000 users). Even with dedicated staff, it may not be possible to subjectively review and judge multiple streams of raw sensor data to determine user satisfaction of a large number of users.

Another example drawback of the conventional approach is that, in the future, reading and understanding raw sensor data captured via more sophisticated wearable and IoT devices may become more difficult.

One embodiment of the present invention provides a system and method for automatically determining user satisfaction in a scalable manner for all users without causing engagement fatigue from multiple surveys. If user satisfaction is accurately determined, a potential decline in user satisfaction over time may be addressed pre-emptively before a user disengages from completing a given wellness plan. For example, a wellness manager may increase communication with a user, offer more encouragement, or request that the user's family and friends lend more support to the user, thereby increasing the likelihood of the user staying on track to complete a given wellness plan.

Some existing commercial software products offer the ability to track user physical activity data over time. For example, some mobile applications may obtain user physical activity data captured by sensors on a mobile device (e.g., a smartphone) or a wearable device (e.g., a smartwatch). Data from such existing commercial software products may be visualized as trend charts, thereby allowing medical wellness managers to determine if a user is adhering to a wellness plan. None of the existing commercial software products, however, calibrate user physical activity data with additional ground-truth satisfaction data and other contextual cues. Further, none of the existing commercial software products integrate with medical alert and medical response systems.

One embodiment provides a framework for automatically determining user satisfaction with a wellness plan as a whole in addition to a wellness plan's different aspects. A determination of user satisfaction may be based on a statistical machine learning model that processes performance-related data (e.g., measurable physical activity), social/context-related data, and ground-truth user satisfaction data of multiple users. In one embodiment, if a user has declining or low user satisfaction with a wellness plan, the framework automatically alerts a wellness manager, a family member, and/or a friend of the user of the declining or low user satisfaction. In one embodiment, if a user is dissatisfied with a wellness plan, the framework automatically generates one or more personalized corrective recommended actions for the user or a wellness manager to improve user satisfaction. In one embodiment, the framework automatically adapts execution based on application scenario.

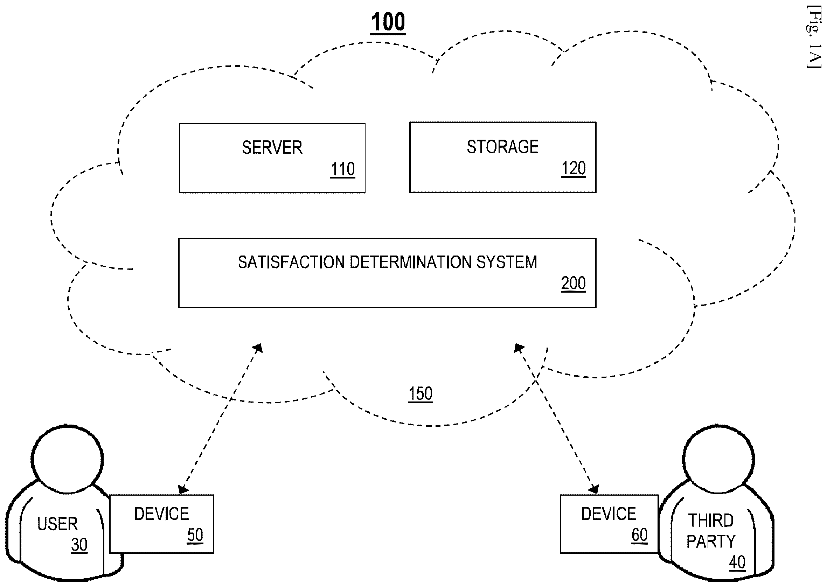

FIG. 1A illustrates an example computing framework 100 for implementing a satisfaction determination system 200, in one or more embodiments. The computing framework 100 comprises an online secure cloud computing environment 150 comprising computation hardware such as, but not limited to, one or more server devices 110 and one or more storage devices 120. One or more applications may execute/operate on the computation hardware of the cloud computing environment 150. In one embodiment, the applications include one or more components of a satisfaction determination system 200 for automatically determining and responding to user satisfaction of one or more users 30 with one or more wellness plans.

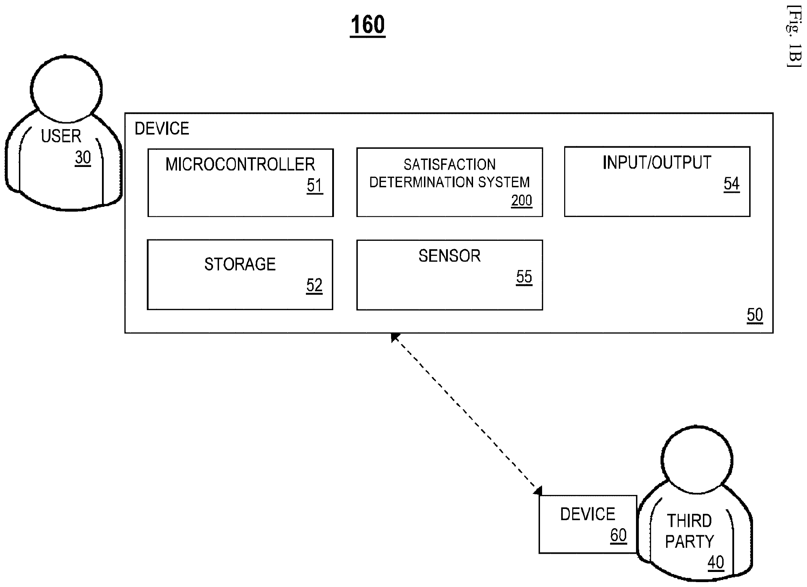

The satisfaction determination system 200 is configured to exchange data with one or more devices (e.g., over a connection such as WiFi, or a cellular data connection). For example, a device 50 may be carried/worn/utilized by a user 30 participating in a wellness plan. The device 50 utilized by the user 30 may comprise at least one of the following: (1) one or more on-board sensors 55 (FIG. 1B) for capturing raw sensor data (e.g., an accelerometer for measuring physical acceleration of the user 30 on independent axes, a GPS receiver for determining a geolocation of the user 30, a heartrate sensor, etc.), and (2) an on-board microcontroller 51 (FIG. 1B) for performing computation on the raw sensor data captured (e.g., computing the number of steps the user 30 has taken, inferring physical activity of the user 30, such as walking, running, biking, etc.). As described in detail later herein, data captured by the device 50 (e.g., raw sensor data) may be forwarded to the satisfaction determination system 200 for determining user satisfaction of the user 30 with the wellness plan and/or an adjustment to the wellness plan.

If the user 30 utilizes multiple devices 50, the devices 50 may work separately or in tandem. Data collected by one device 50 (e.g., a smartwatch) of the user 30 may be synced to another device 50 (e.g., a smartphone) of the user 30 at a later time. For example, a smartphone carried by a user 30 may be paired with one or more other devices 50 located in a car, home, and/or office of the user 30. The satisfaction determination system 200 analyzes data collected by an individual device 50, or through paired devices 50, to determine user satisfaction of the user 30 with the wellness plan and/or an adjustment to the wellness plan.

A different device 60 may be carried/worn/utilized by a third party member 40 associated with the user 30. The satisfaction determination system 200 may send an alert to the device 60 of the third party member 40 to notify the third party member 40 of the determined user satisfaction of the user 30 with the wellness plan. The alert may prompt/invoke the third party member 40 to take action, such as directly contacting the user 30 or changing/adapting one or more aspects of the wellness plan to improve user satisfaction.

FIG. 1B illustrates another example computing framework 160 for implementing a satisfaction determination system 200, in one or more embodiments. In another embodiment, one or more components of the satisfaction determination system 200 resides on a device 50 of a user 30 participating in a wellness plan. The satisfaction determination system 200 may execute/operate on computation hardware of the device 50 such as, but not limited to, an on-board microcontroller 51 and one or more storage units 52.

The satisfaction determination system 200 is configured to exchange data with one or more other devices (e.g., over a connection such as WiFi, or a cellular data connection). For example, the satisfaction determination system 200 may send an alert to a device 60 carried/worn/utilized by a third party member 40 associated with the user 30.

In one embodiment, the device 50 comprises one or more input/output (I/O) modules 54 integrated in or coupled to the device 50, such as a keyboard, a keypad, a touch interface, a display screen, etc.

In one embodiment, operation of the satisfaction determination system 200 may be divided into two different operating phases ? a training phase and a determination phase. As described in detail later herein, in the training phase, the satisfaction determination system 200 is configured to: (1) collect training data from multiple users participating in one or more wellness plans, and (2) based on the collected training data, train a statistical machine learning model ("statistical model") for use in determining user satisfaction with a wellness plan. In the determination phase, the satisfaction determination system 200 is configured to: (1) collect data from a user participating in a wellness plan, (2) based on the collected data and a learned statistical model (e.g., a statistical model learned during the training phase), determine satisfaction of the user with the wellness plan, and (3) based on the satisfaction determined, selectively send an alert to a third party member associated with the user and/or selectively generate a recommendation for improving the satisfaction of the user with the wellness plan.

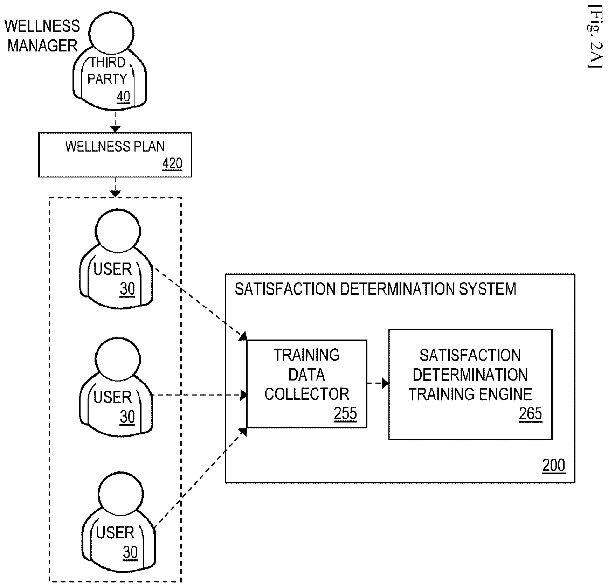

FIG. 2A illustrates one or more example components of the satisfaction determination system 200 utilized in the training phase, in one or more embodiments. The satisfaction determination system 200 comprises at least one of the following components utilized in the training phase: a training data collector 255, a satisfaction determination training engine 265, and a combination thereof.

As stated above, a user 30 may participate in a wellness plan 420. A wellness manager 40 may utilize remote user monitoring to keep track of the progress of the user 30 towards a desired goal of the wellness plan 420. To facilitate remote user monitoring, the user 30 may utilize one or more devices 50 (FIG. 1A), such as a smartphone, a wearable device (e.g., a smartwatch, a sensor patch, etc.), and/or an IoT device (e.g., a home sensor) for tracking/capturing contextual information and/or physical activity of the user 30. Some of the data tracked/captured by the one or more devices 50 may be done in a real-time manner.

In a training phase, the training data collector 255 is configured to collect training data from multiple users 30 participating in one or more wellness plans. The collected training data comprises performance-related data (e.g., raw sensor data captured by sensors) and social/context-related data (e.g., data from online services, such as social networking sites, etc.) associated with the multiple users 30 such as, but not limited to, medical health records, measurable physical activity (e.g., data indicative of a number of steps taken, time spent sleeping, etc.), social networking/online usage (e.g., comments posted on online forums/message boards, online purchases), contextual cues (e.g., weather conditions, etc.), ground-truth user satisfaction (e.g., user responses to surveys inquiring about user satisfaction with a given wellness plan on a well-understood scale, such as between 1 and 5 stars), etc. Based on the collected training data, the training data collector 255 maintains, on at least one storage device (e.g., a storage device 120 in FIG. 1A or a storage unit 52 in FIG. 1B), one or more databases comprising historical data across the multiple users 30. In one embodiment, the training data collector 255 provides an application programming interface (API) that facilitates collection of training data from different devices 50 of the multiple users 30 and different data sources.

In one embodiment, the training data collector 255 is configured to collect ground-truth user satisfaction data from a user 30 with respect to an entirety of a wellness plan or specific, granular aspects of the wellness plan.

In the training phase, the satisfaction determination training engine 265 is configured to train, based on the collected training data, a statistical model for mapping performance-related and/or social/context-related data to ground-truth user satisfaction data. A learned statistical model resulting from the training phase may be used in the determination phase for determining the satisfaction of an individual user 30 with a wellness plan.

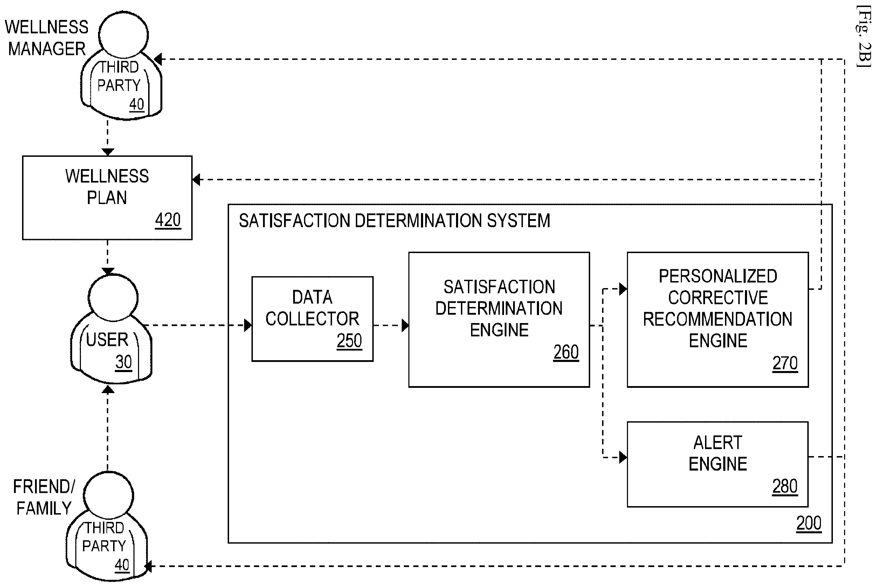

FIG. 2B illustrates one or more example components of the satisfaction determination system 200 utilized in the determination phase, in one or more embodiments. The satisfaction determination system 200 comprises at least one of the following components utilized in the determination phase: a data collector 250, a satisfaction determination engine 260, a personalized corrective recommendation engine 270, an alert engine 280, and a combination thereof.

In the determination phase, the data collector 250 is configured to collect data from a user 30 participating in a wellness plan 420. The collected data comprises performance-related data (e.g., raw sensor data captured by sensors) and social/ context-related data (e.g., data from online services, such as social networking sites, etc.) associated with the user 30 such as, but not limited to, medical health records, measurable physical activity (e.g., data indicative of a number of steps taken, time spent sleeping, etc.), social networking/online usage (e.g., comments posted on online forums/message boards, online purchases), contextual cues (e.g., weather conditions, etc.), etc. Based on the collected data, the data collector 250 maintains, on at least one storage device (e.g., a storage device 120 in FIG. 1A or a storage unit 52 in FIG. 1B), one or more databases comprising data for the user 30. As described in detail later herein, the collected data may be used for determining the satisfaction of the user 30 with a wellness plan. In one embodiment, the data collector 250 provides an API that facilitates collection of data from different devices 50 of the user 30 and different data sources.

In the determination phase, the satisfaction determination engine 260 is configured to determine satisfaction of the user 30 with a wellness plan and/or specific aspects of the wellness plan based on the collected data and a learned statistical model (e.g., a statistical model learned during the training phase). The satisfaction determination engine 260 may generate a report that explains how the satisfaction is determined.

In one embodiment, one or more components of the satisfaction determination system 200 may execute/operate on the computation hardware of the cloud computing environment 150, while one or more remaining components of the satisfaction determination system 200 may execute/operate on a device 50 of a user 30. For example, one or more components utilized in the training phase (e.g., the training data collector 255 and/or the satisfaction determination training engine 265) may run on a server device 110 of the cloud computing environment 150, while one or more components utilized in the determination phase (e.g., the data collector 250, the satisfaction determination engine 260, the personalized corrective recommendation engine 270, and/or the alert engine 280) may run on a device 50 of a user 30. As another example, the satisfaction determination engine 260, the personalized corrective recommendation engine 270, and/or the alert engine 280 may run on a server device 110 of the cloud computing environment 150. As yet another example, the satisfaction determination engine 260 and/or the personalized corrective recommendation engine 270 may run on a device 50 of a user 30 to preserve user privacy (i.e., sensitive user data resides on the device 50 instead of a remote server device 110).

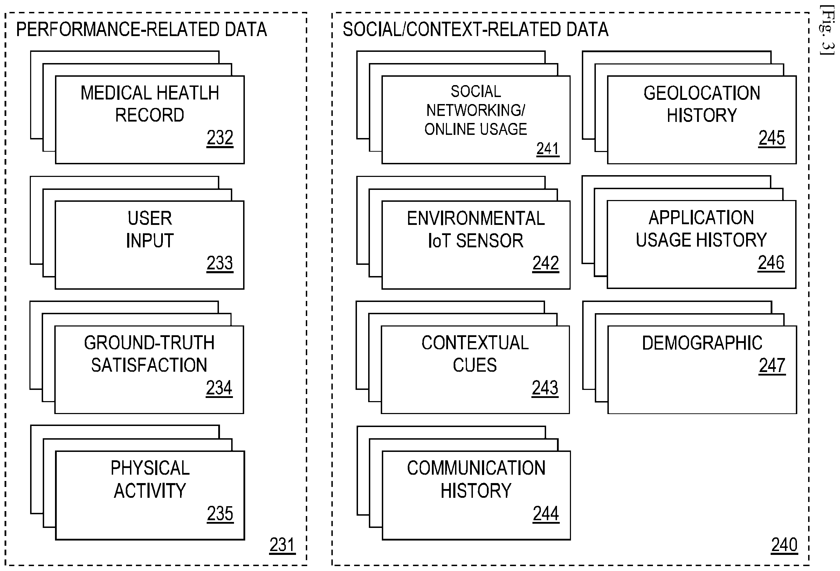

FIG. 3 illustrates an example of different types of data collected by the satisfaction determination system 200, in one or more embodiments. In one embodiment, the training data collector 255 and/or the data collector 250 maintains, on at least one storage device (e.g., a storage device 120 in FIG. 1A or a storage unit 52 in FIG. 1B), at least one database comprising a first collection 231 of performance-related data records and a second collection 240 of social/context-related data records. The first collection 231 comprises different types of performance-related data records such as, but not limited to, the following: (1) data records 232 comprising medical health records, (2) data records 233 comprising user input data, (3) data records 234 comprising ground-truth user satisfaction data, and (4) data records 235 comprising measurable physical activity data.

The second collection 240 comprises different types of social/context-related data records such as, but not limited to, the following: (1) data records 241 comprising social networking/online usage data, (2) data records 242 comprising environmental IoT sensor data, (3) data records 243 comprising contextual cues data, (4) data records 244 comprising communication history data, (5) data records 245 comprising geolocation history data, (6) data records 246 comprising application usage history data, and (7) data records 247 comprising demographic data.

A medical health record for a user 30 may include information such as, but is not limited to, age of the user 30, gender of the user 30, weight of the user 30, height of the user 30, body mass index (BMI) of the user 30, and one or more medical conditions of the user 30 (e.g., obesity, hypertension, diabetes, cardiac rehabilitation, etc.). The training data collector 255 and/or the data collector 250 may obtain all or a portion of the medical health record via several means such as, but not limited to, the user 30 manually entering the information via a software user interface running on a device 50 carried/worn/utilized by the user 30, or a secure online REST call between the satisfaction determination system 200 and a remote server of a medical provider that provides medical services to the user 30.

For one or more items that cannot be inferred automatically, a user 30 may manually enter user input data via a software user interface running on a device 50 carried/ worn/utilized by the user 30. The training data collector 255 and/or the data collector 250 may obtain the user input data from the device 50. The user input data may include, but is not limited to, one or more types of food consumed during a most recent meal of the user 30, number of calories in the meal, whether or not the user 30 has taken medication, user preferences of the user 30, etc.

Physical activity data for a user 30 may include information such as, but not limited to, step count (i.e., number of steps the user 30 has taken throughout a day), exercise performed (e.g., walking, running, biking, etc.), duration of the exercise performed, instantaneous heart rate and peak heart rate during the day, amount of sleep, quality of sleep (e.g., duration of REM sleep), etc. The physical activity data may be automatically captured by one or more bio-sensors of a device 50 carried/worn/utilized by the user 30. The training data collector 255 and/or the data collector 250 may obtain the physical activity data from the device 50.

Environmental IoT sensor data for a user 30 comprises information relating to an environment of the user 30 such as, but not limited to, different appliances the user 30 interacts with, television programs the user 30 frequently watches, rooms that the user 30 spends the most time in while at home, etc. The environmental IoT sensor data may be automatically captured by one or more IoT sensors. The training data collector 255 and/or the data collector 250 may obtain the environmental IoT sensor data from the IoT sensors.

Contextual cues data for a user 30 may include information such as, but not limited to, weather in the vicinity of the user 30, current date or holiday season, personal/ business calendar of the user 30, etc. The training data collector 255 and/or the data collector 250 may obtain the contextual cues data from at least one device 50 carried/ worn/utilized by the user 30 and/or at least one online data source.

Communication history data for a user 30 comprises information relating to one or more communications exchanged between the user 30 and at least one third party member 40 (e.g., a wellness manager, a family member, and/or a friend). For example, the communication may include, but are not limited to, e-mails, phone calls, and text messages relating to a wellness plan for the user 30. The communication history data may include content of each communication exchanged and/or metadata associated with the communication (e.g., time/data of the communication). The training data collector 255 and/or the data collector 250 may obtain the communication history data from at least one device 50 carried/worn/utilized by the user 30.

Social networking/online usage data for a user 30 comprises information relating to one or more online activities of the user 30, such as social networking and online shopping. For example, the social networking/online usage data may include, but is not limited to, one or more comments posted on one or more online forums/message boards, one or more associations the user 30 may have with other people, one or more websites the user 30 visits online, one or more items that the user 30 purchases via an online shopping portal, one or more product ratings the user 30 has given to one or more items, etc. The training data collector 255 and/or the data collector 250 may obtain the social networking/online usage data from at least one device 50 carried/ worn/utilized by the user 30.

Geolocation history data for a user 30 comprises information indicative of one or more locations the user 30 is currently at or was previously at. The geolocation history data may be in the form of latitude and longitude geocoordinates or location names (e.g., mailing address or landmark). The geolocation history data may be automatically captured by one or more sensors of a device 50 carried/worn/utilized by the user 30. The training data collector 255 and/or the data collector 250 may obtain the geolocation history data from the device 50.

Application usage history data for a user 30 may include information such as, but not limited to, one or more software applications the user 30 has installed, one or more software applications the user 30 has interacted with, multimedia the user 30 has played, etc. The training data collector 255 and/or the data collector 250 may obtain the application usage history data from a device 50 carried/worn/utilized by the user 30.

Demographic data for a user 30 may include information such as, but not limited to, an education level of the user 30, a new worth of the user 30, one or more preferred shopping locations, etc. The training data collector 255 and/or the data collector 250 may automatically collect/infer the demographic data from other types of data collected for the user 30.

Ground-truth user satisfaction data for a user 30 comprises information indicative of a satisfaction of the user 30 with a wellness plan and/or specific aspects of the wellness plan. As described in detail later herein, a software application associated with the satisfaction determination system 200 may be downloaded to/loaded onto a device 50 carried/worn/utilized by the user 30. The software application is configured to generate a software user interface including one or more survey questions for display on the device 50. The user 30 may interact with the software user interface to provide a response/reply indicative of his/her satisfaction with the wellness plan and/or the specific aspects of the wellness plan.

To prevent engagement fatigue, the collection of ground-truth user satisfaction data for the user 30 may not continue indefinitely. In one embodiment, survey questions are occasionally presented to the user 30 during a time period starting from the beginning/ commencement of the wellness plan. The initial time period and the frequency of presenting survey questions may be set by a wellness manager 40 tasked with monitoring/supervising progress of the user 30 with the wellness plan. For example, the initial time period may be 6 weeks, the frequency of presenting survey questions may be once a week, and time of day when the survey questions are presented may be randomly chosen to be between 10:00 AM and 2:00 PM. After the initial time period has elapsed, the satisfaction determination system 200 may determine satisfaction of the user 30 without prompting the user 30 with additional survey questions.

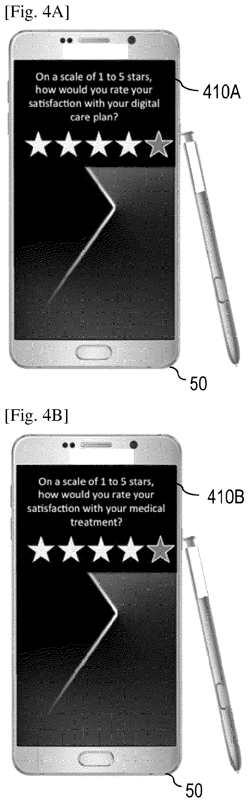

FIGS. 4A-4C illustrate different example software user interfaces displayed on a device 50 for collection of ground-truth user satisfaction data, in one or more embodiments. Specifically, FIG. 4A illustrates an example software user interface 410A displayed on a smartphone 50, in one or more embodiments. As shown in FIG. 4A, the software user interface 410A prompts a user 30 carrying/utilizing the smartphone 50 to rate, on a scale of 1 to 5 stars, his/her overall satisfaction with a given wellness plan (e.g., digital care plan). In one embodiment, 5 stars indicates a maximum/highest satisfaction level/score the user 30 can specify. The user 30 may enter a response/reply utilizing an I/O device of the smartphone 50 (e.g., tapping, using a stylus or his/her finger, a touch screen of the smartphone 50 to highlight one or more of the stars displayed). The training data collector 255 records the response/reply entered as ground-truth user satisfaction data for the user 30.

In another embodiment, the satisfaction may be specified using a different scale (e.g., between integers 1 to 10, between floating-point numbers 0.0 and 1.0, between two choices such as "dissatisfied" and "satisfied").

FIG. 4B illustrates another example software user interface 410B displayed on a smartphone 50, in one or more embodiments. As shown in FIG. 4B, the software user interface 410B prompts a user 30 carrying/utilizing the smartphone 50 to rate, on a scale of 1 to 5 stars, his/her satisfaction with an aspect of a given medical plan, such as medical treatment received.

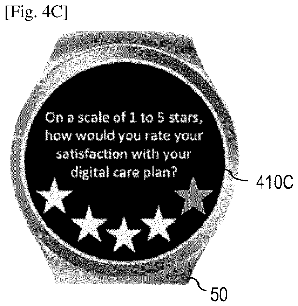

FIG. 4C illustrates yet another example software user interface 410C displayed on a smartwatch 50, in one or more embodiments. As shown in FIG. 4C, the software user interface 410C prompts a user 30 wearing the smartwatch 50 to rate, on a scale of 1 to 5 stars, his/her overall satisfaction with a given wellness plan (e.g., digital care plan). The user 30 may enter a response/reply utilizing an I/O device of the smartwatch 50 (e.g., tapping, using his/her finger, a touch screen of the smartwatch 50 or turning a circular bezel of the smartwatch 50 to highlight one or more of the stars displayed).

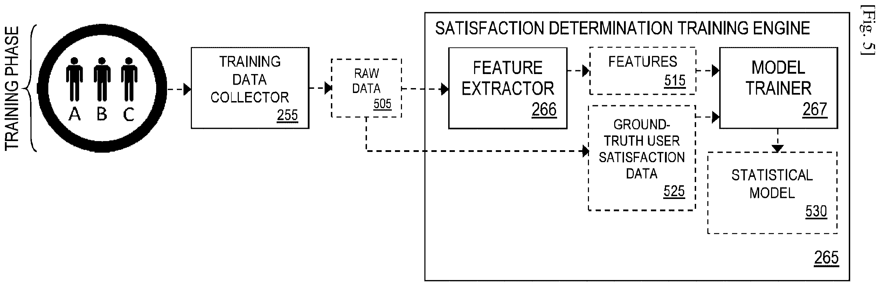

FIG. 5 illustrates one or more examples processes performed by the satisfaction determination system 200 during the training phase, in one or more embodiments. The satisfaction determination training engine 265 comprises at least one of the following: (1) a feature extractor 266, and (2) a machine learning model trainer 267. In the training phase, the training data collector 255 collects training data by obtaining data from multiple users 30, such as user A, user B, and user C. The collected training data comprises raw data 505 such as, but not limited to, performance-related data, social/ context-related data, and ground-truth user satisfaction data 525 for each user 30. The feature extractor 266 is configured to extract multiple features 515 for each user 30 from the raw data 505. An extracted feature for a user 30 may comprise either data in a raw form (e.g., user A jogged 12,000 steps on April 10, 2016 between 1:30PM and 2:15PM with a peak heart rate of 101 beats per minute) or a specific range bin in which the user 30 may be placed (e.g., a 45-year-old user may be placed into a range bin for 40-50 year old users).

The model trainer 267 is configured to train, based on each extracted feature for each user 30, a statistical model 530 that maps multiple extracted features for each user 30 to ground-truth user satisfaction data 525 for the user 30.

The model trainer 267 may train the statistical model 530 on a server device 110 of the cloud computing environment 150 or a device 50 of a user 30.

In one embodiment, the model trainer 267 may be implemented using a software programming language or obtained from an off-the-shelf trainer software package. In one embodiment, different algorithms may can be used for supervised machine learning classification such as, but not limited to, Logistic Regression, Decision Tree, Naive Bayes, Support Vector Machine, or Artificial Neural Network.

In one embodiment, the statistical model 530 may be re-built on an as-needed basis. In one embodiment, depending on the algorithm used for supervised machine learning classification, the statistical model 530 may be updated online after new data is collected by the training data collector 255.

In one embodiment, the collected training data may include communication history data for each user 30, such as emails and text messages exchanged between the user 30 and a third party member 40 (e.g., a wellness manager, a family member, a friend). For example, the training data collector 255 may obtain communication history data from one or more communication software applications executing on one or more devices 50 of a user 50 (e.g., an email software client, a text messaging application, a chat client, and other types of communication software applications). The model trainer 267 may learn to determine user sentiment of each user 30 based on the communication history data. For example, a user sentiment may be represented on a scale ranging from "positive sentiment", "neutral", to "positive sentiment". The user sentiment may be computed by applying one or more algorithms for analyzing sentiments, such as counting known positive-sentiment and negative-sentiment adjectives and nouns in emails and text messages and computing a net sentiment, or applying statistical machine learning.

In one embodiment, the collected training data may include social networking/online usage data for each user 30. For example, the training data collector 255 may obtain social networking/online usage data from one or more social networking/web software applications executing on one or more devices 50 of a user 50 (e.g., a web browser, a social networking application, and other types of social networking/web software applications). The training data collector 255 may also obtain social networking/online usage data from one or more mobile payment software applications and/or digital distribution platforms executing on one or more devices 50 of a user 50 (e.g., a mobile payment application for use in purchasing items online or at a physical retail store, an app store/marketplace from which digital items may be purchased, and other types of mobile payment software applications and/or digital distribution platforms). Features extracted from social networking/online usage data for a user 30 may be used in determining satisfaction of the user 30. For example, the training data collector 250 may monitor one or more online postings/reviews associated with an online identification of a user 30 on one or more online sites based on social networking/online usage data for the user 30. If the user 30 posted a negative-sentiment comment or wrote a negative review for a given wellness plan on an online forum/message board/social networking site, satisfaction of the user 30 may be determined. As another example, if the user 30 explicitly states his/her mood on a social networking site, satisfaction of the user 30 may also be determined. As yet another example, the training data collector 250 may monitor one or more purchases of a user 30 based on social networking/online usage data for the user 30. If the user 30 purchases items such as books or off-the-shelf medication to treat depression, satisfaction of the user 30 may also be determined.

In one embodiment, the collected training data may include application usage history data for each user 30. Features extracted from application usage history data for a user 30 may be used in determining satisfaction of the user 30. For example, frequency at which the user 30 interacts with a mobile application for management of a given wellness plan may be tracked to determine whether there is a decline or growth in user engagement with the mobile application. As another example, user interactions with news, games, music, video, and other mobile applications may be tracked to determine whether there is a decline or growth in user interaction.

In one embodiment, the collected training data may include demographic data for each user 30. Features extracted from demographic data for a user 30 may be used in determining satisfaction of the user 30. For example, the satisfaction determination training engine 265 may cluster users 30 together by similar socioeconomic buckets, such that historical data between different users 30 is comparable. Users 30 who have similar education level, net worth, and shopping preferences/habits may be placed into the same socioeconomic bucket. The satisfaction determination training engine 265 may train one statistical model per socioeconomic bucket so that different behaviors that are inherent to different socioeconomic buckets will not result in a poorly-determining statistical model.

In one embodiment, one or more additional features may be derived/computed from raw data 505 for a user 30. These additional features may include, but are not limited to, applying low-pass smoothing filters on noisy time series data, summing time series data over a sliding window, summing step counts into specific weekly and monthly range bins, summing number of times the user 30 has called or emailed a wellness manager 40 in recent weeks, and computing gradient of value changes between 60 days ago and current date.

In one embodiment, the satisfaction determination training engine 265 is configured to determine which of the features extracted/derived has little or no impact in determining user satisfaction. For example, if satisfaction levels for multiple users 30 are similar, the satisfaction determination training engine 265 may determine which of the features extracted/derived has significant variation across the multiple users 30; each feature exhibiting significant variation may be designated as a feature having little to no impact in determining user satisfaction.

In one embodiment, features extracted/derived from raw data 505 for a user 30 may be weighted differently in order to emphasize some features more than other features. The weighting of each feature may be based on context and one or more characteristics/requirements of a given wellness plan. For example, for medical scenarios, features relating to health and fitness may be emphasized with a higher weight. The weighting of each feature may additionally be based on impact of the feature in determining user satisfaction. For example, if a feature has little to no impact in determining user satisfaction, the feature may be de-emphasized with a lower weight.

In the training phase, a statistical model 530 may be trained based on training data collected from a set of users 30 enrolled in a particular program. A learned statistical model 530 resulting from the training phase may be automatically applied, in the determination phase, for many users 30 enrolled in the same or similar program in a scalable manner, thereby removing the need for a wellness manager 40 making a subjective judgment for each user 30 based on a review of his/her data.

For example, in the training phase, a statistical model 530 may be trained based on training data collected from a set of users 30 exhibiting the following characteristics: (i) male, (ii) 30-35 years old, (iii) 31-35 BMI, (iv) participates in a cardiac rehabilitation program, (v) exhibits a decline in average step count per week over the course of 60 days during summer months, (vi) exhibits an increase in negative-sentiment communications sent to a wellness manager 40 in recent weeks, and (vii) indicates "very dissatisfied" in response to a survey inquiring about user satisfaction with the program.

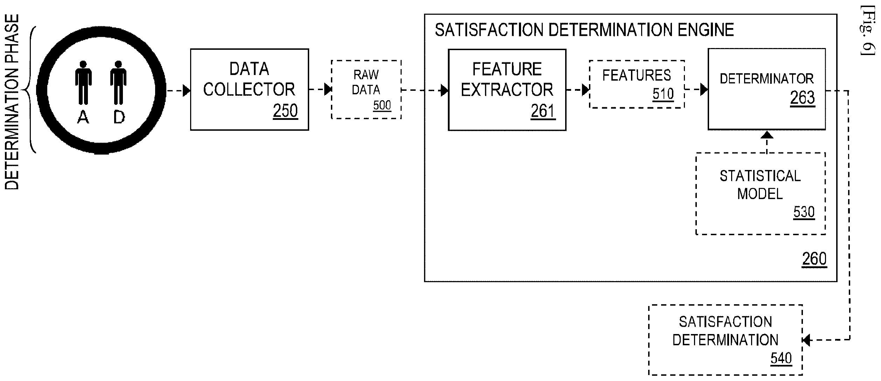

FIG. 6 illustrates one or more examples processes performed by the satisfaction determination system 200 during the determination phase, in one or more embodiments. In the determination phase, the satisfaction determination engine 260 applies the learned statistical model 530 (e.g., the statistical model 530 learned during the training phase) to determine satisfaction of a user 30 with a wellness plan and/or specific aspects of the wellness plan (e.g., satisfaction of user A who contributed to the training data, satisfaction of user D who did not contribute to the training data).

In one embodiment, the satisfaction determination engine 260 is configured to determine user satisfaction of a user 30 on-demand or at some periodic interval (e.g., once a day or once a week). For example, as shown in FIG. 6, in the determination phase, the satisfaction determination engine 260 may determine user satisfaction of user A (who contributed to the training data obtained during the training phase) and user D (who did not contribute to the training data obtained during the training phase). As such, a user 30 involved in a wellness plan that spans many months (e.g., 6 months) may be asked survey questions once a week for an initial duration of two months; thereafter, a resulting learned statistical model 530 may be applied to determine user satisfaction of the user 30. This obviates the need to continually prompt the user 30 to answer survey questions. Further, the statistical model 530 may be applied to other users 30 who were not previously asked survey questions (e.g., user D). Those skilled in the art of this invention will understand that following machine learning best practices may allow the trained model to be generalizable to produce accurate results even for new data that has previously not been encountered.

The collected data comprises raw data 500 such as, but not limited to, performance-related data and/or social/context-related data for each user 30. The feature extractor 261 is configured to extract multiple features 510 for each user 30 from the raw data 500. The features 510 extracted correspond to the same features extracted during the training phase.

The satisfaction determination engine 260 further comprises a determinator 263. In the determination phase, for each user 30, the determinator 263 is configured to apply a learned statistical model 530 to determine satisfaction determination information 540 for the user 30. Specifically, the determinator 263 reads features 510 extracted for the user 30, performs a computation appropriate/suitable for a machine learning algorithm being applied, and outputs satisfaction determination information 540 for the user 30. The satisfaction determination information 540 comprises a determination of user satisfaction of the user 30 with a wellness plan and/or specific aspects of the wellness plan. The determination may be a probability distribution over different user satisfaction levels (e.g., the user 30 is 5% "very dissatisfied", 5% "somewhat dissatisfied", 10% "neutral", 70% "somewhat satisfied", and 10% "very satisfied") or a single determination (e.g., the user 30 is "satisfied").

For example, the satisfaction determination engine 260 may apply a statistical model 530 trained based on data collected from a set of users 30 exhibiting the following characteristics: (i) male, (ii) 30-35 years old, (iii) 31-35 BMI, (iv) participates in a cardiac rehabilitation program, (v) exhibits a decline in average step count per week over the course of 60 days during summer months, (vi) exhibits an increase in negative-sentiment communications sent to a wellness manager 40 in recent weeks, and (vii) indicates "very dissatisfied" in response to a survey inquiring about user satisfaction with the cardiac rehabilitation program. Based on the statistical model 530, the satisfaction determination engine 260 may determine user satisfaction of every user 30 enrolled in the same cardiac rehabilitation program. For example, a user 30 who exhibits similar characteristics as above has a 75% chance of being "very dissatisfied".

The satisfaction determination engine 260 is configured to generate a report including an explanation as to how a determination included in satisfaction determination information 540 for a user 30 was determined. The report may provide a third party member 40 with insight as to why the user 30 is dissatisfied. The explanation may be based on an internal structure of the statistical model 530. In one embodiment, if a decision tree is utilized, a path of edges from a root of the tree to a leaf of the tree that corresponds to the determination may be traced; as each edge is an outcome of a decision at an inner tree node, the explanation may be derived from concatenating outcomes of decisions at inner tree nodes together. In another embodiment, one or more algorithms for feature selection may be utilized to calculate the most important features that lead to the lowest error; the explanation is based on these important features.

In one embodiment, the satisfaction determination engine 260 is configured for fine-grained user satisfaction determination. Specifically, the satisfaction determination engine 260 is configured to determine user satisfaction of a user 30 with respect to an entirety of a wellness plan or specific, granular aspects of the wellness plan. Examples of specific, granular aspects of a wellness plan may include, but are not limited to, intensity of aerobic exercise, intensity of anaerobic exercise, medication dosage, quality of software and hardware used for remote patient monitoring, and quality of a wellness manager. A user 30 may have different user satisfactions for different aspects of a wellness plan.

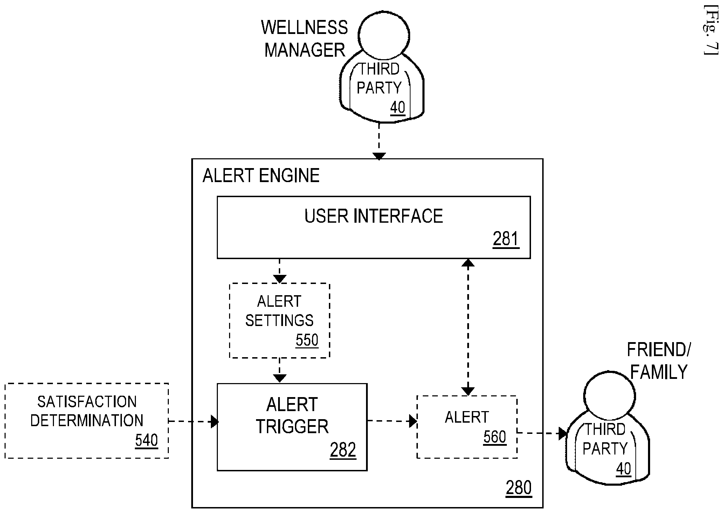

FIG. 7 illustrates an example alert engine 280 in detail, in one or more embodiments. The alert engine 280 comprises an alert trigger unit 282 configured to: (1) receive satisfaction determination information 540 for a user 30 from the satisfaction determination engine 260, (2) determine whether a prediction of user satisfaction included in the satisfaction determination information 540 is low based on one or more pre-programmed alert settings 550 (e.g., whether the prediction of user satisfaction is less than a pre-programmed threshold value), and (3) in response to determining the prediction of user satisfaction is low, send an alert to at least one third party member 40 (e.g., a wellness manager, a family member, or a friend) associated with the user 30.

The alert engine 280 further comprises a user interface generator 281 configured to generate one or more software user interfaces that may be accessed by one or more third party members 40.

As one example, a third party member 40, such as a wellness manager, may utilize the software user interface to pre-program one or more alert settings 550. In one embodiment, the alert settings 550 comprise a rule that specifies when an alert is triggered. Specifically, the rule defines a threshold value used in determining whether user satisfaction is low, wherein the threshold value is on the same scale as the determination. For example, assume the scale utilized for determining user satisfaction is a 5-level scale including the following satisfaction levels in ascending order: "very dissatisfied", "somewhat dissatisfied", "neutral", "satisfied", and "very satisfied". A third party member 40 (e.g., a wellness manager) may specify a rule defining that an alert should be triggered (i.e., an alert sent to at least one third party member 40) if the prediction is less than/below a pre-programmed threshold value of "neutral" (i.e., an alert is triggered if the determination is "very dissatisfied" or "somewhat dissatisfied"). In another embodiment, the rule may instead define that an alert should be triggered if the determination is at or above a pre-programmed threshold value.

In one embodiment, a software user interface generated by the user interface generator 281 comprises a webpage that allows a third party member 40 (e.g., a wellness manager) to pre-program one or more alert settings 55. For example, the webpage may comprise at least one of the following: (1) a first textbox for receiving a pre-programmed threshold value, and (2) a set of mutually-exclusive radio buttons for specifying whether a prediction of user satisfaction should be above/more, the same as, or below/less than the pre-programmed threshold value to trigger an alert. As another example, the webpage may comprise a graphical slider that the third party member 40 may interact with to specify a pre-programmed threshold value.

In one embodiment, a rule specifying when an alert is triggered may be stored and evaluated as a predicate trigger inside a SQL database; an alert is sent as soon as any prediction of user satisfaction satisfies the predicate trigger.

As stated above, each third party member 40 alerted may utilize a software user interface generated by the user interface generator 281 to contact the user 30. A third party member 40 may utilize the software user interface to provide encouragement, advice, and/or admonishment to the user 30 in order to guide the user 30 back on track with a wellness plan.

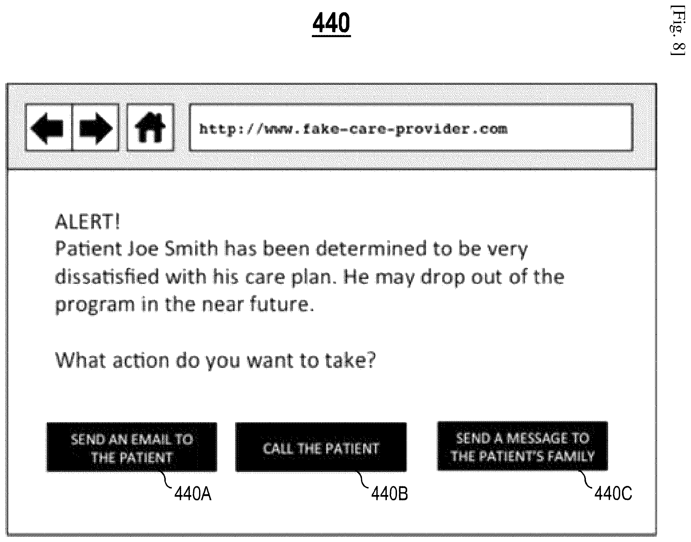

FIG. 8 illustrates an example software user interface 440 providing an alert, in one or more embodiments. The software user interface 440 comprises a webpage dashboard accessible by a third party member 40. The webpage dashboard includes the name and predicted user satisfaction of a user 30 associated with the third party member 40. The webpage dashboard may further comprise a widget that upon actuation by the third party member 40 invokes another software component. For example, the webpage dashboard may comprise at least one of the following widgets: (1) a first graphical button 440A to invoke an email software client that allows the third party member 40 to write and send an email to the user 30, (2) a second graphical button 440B to invoke a voice-over-IP telecommunications program that allows the third party member 40 to make a phone call to the user 30, and (3) a third graphical button 440C to invoke sending of an automated message to another third party member 40 (e.g., a family member, friend) to provide information regarding the determined user satisfaction of the user 30. As another example, an alert sent to a third party member 40 may be via an email or a mobile device text message. In a another embodiment, the software user interface 440 may be part of a native mobile application running on a device 60 (e.g., a tablet computer) of a third party member 40.

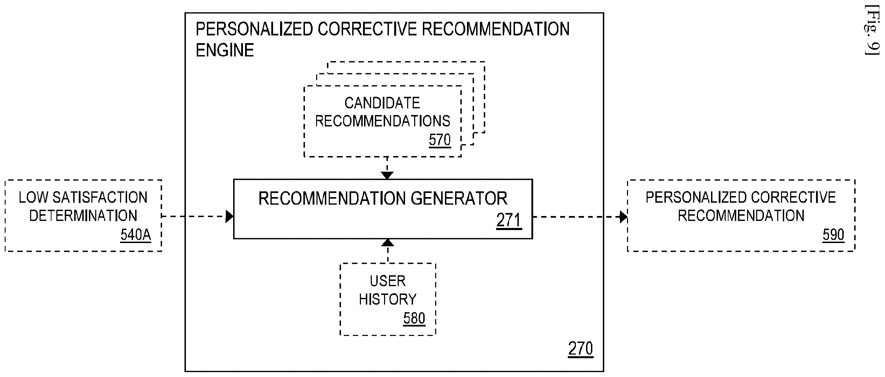

FIG. 9 illustrates an example personalized corrective recommendation engine 270 in detail, in one or more embodiments. The recommendation engine 270 comprises a recommendation generator 271 configured to: (1) receive, from the satisfaction determination engine 260, a determination 540A for a user 30 indicative of low user satisfaction with a wellness plan, and (2) automatically recommend a course of action by generating one or more corrective actionable recommendations 590 that may increase user satisfaction of the user 30. A corrective actionable recommendation 590 may comprise a non-medical recommendation (e.g., exercising at a different park) or a medical recommendation (e.g., prescribing new medication, changing medication dosage, reducing exercise strain on a knee). Examples of different types of corrective actionable recommendations 590 include, but are not limited to, medication dosage (e.g., reducing a medication from 40mg to 10mg), diet or daily caloric intake (e.g., eating tofu instead of meat, or reducing intake to 1200 calories/day), mode of exercise (e.g., running or bicycling), intensity of exercise (e.g., increasing heart rate from 90 bpm to 100 bpm), frequency of exercise (e.g., increasing exercise from twice per week to four times per week), location of exercise (e.g., jogging in a park with dedicated jogging trails), etc.

In one embodiment, the satisfaction determination system 200 automatically integrates a non-medical recommendation into the wellness plan. In one embodiment, the satisfaction determination system 200 presents a medical recommendation to a third party member 40 (e.g., a wellness manager) for review and approval before the satisfaction determination system 200 integrates the medical recommendation into the wellness plan.

In one embodiment, the recommendation generator 271 determines one or more corrective actionable recommendations 590 that may increase user satisfaction of the user 30 based on user history data 280. The user history data 280 is indicative of one or more actions the user 30 took in the past that subsequently improved/increased his/her user satisfaction; the corrective actionable recommendations 590 determined may be similar to these past actions.

In another embodiment, the recommendation generator 271 utilizes a collaborative filtering approach to determine one or more corrective actionable recommendations 590 that may increase user satisfaction of the user 30. For example, the corrective actionable recommendations 590 determined may be based on a set of candidate recommendations 570 indicative of actions other users 30 similar to the user 30 took in the past that subsequently improved/increased their user satisfaction; the corrective actionable recommendations 590 determined may be similar to these past actions. As another example, the corrective actionable recommendations 590 determined may be based on a set of candidate recommendations 570 indicative of actions other users 30 satisfied with a given wellness plan and similar to the user 30 took in the past; the corrective actionable recommendations 590 determined may be based on differences between actions of the user 30 and these past actions of the other users 30.

In yet another embodiment, the recommendation generator 271 determines one or more corrective actionable recommendations 590 that may increase user satisfaction of the user 30 by balancing out activities the user 30 has recently performed in view of the overall activity goal identified in a given wellness plan. In yet another embodiment, the recommendation generator 271 recommends a best activity for the user 30 to perform to maintain/improve user satisfaction of the user 30. For example, if the user 30 went swimming already earlier in the day, a corrective actionable recommendation 590 may be for the user 30 to jog later in the evening instead of recommendation of swimming again. As another example, based on calendar/schedule information of the user 30, the satisfaction determination system 200 may know that the user 30 has done some activity/exercise already (e.g., swimming) and may suggest another activity/ exercise. In another example, if the user 30 drives a car more than one hour before exercise, the satisfaction determination system 200 may scale down an amount of exercise recommended to the user 30 or recommend a change of exercise program from arms to weights to heal or recover from stiffness caused by driving. As yet another example, based on data from location/GPS sensors, the satisfaction determination system 200 may recognize presence/absence of spacious factors (e.g., area, air condition, downhill direction, etc.) and make a recommendation accordingly.

In one embodiment, the recommendation generator 271 analyzes a user satisfaction of the user 30 to determine a best mode and intensity of exercise for the user 30 (e.g., running 1km, 1.5km, or 3km based on the user satisfaction).

In one embodiment, a corrective actionable recommendation 590 comprises a recommendation for the user 30 to participate in a different wellness plan.

A corrective actionable recommendation 590 may be presented to a user 30 via a mobile application running on a device 50 carried/worn/utilized by the user 30.

Each corrective actionable recommendation 590 may be applicable to a specific, granular aspect of the wellness plan. The recommendation generator 271 is configured to generate a corrective actionable recommendation 590 that addresses user dissatisfaction with a specific, granular aspect of the wellness plan.

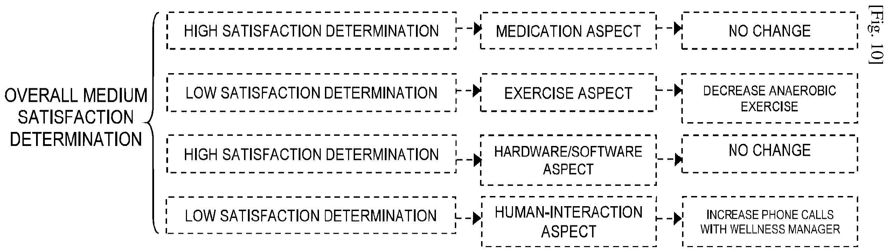

FIG. 10 illustrates an example overall user satisfaction score with a wellness plan based on user satisfaction with specific, granular aspects of the wellness plan, in one or more embodiments. The satisfaction determination system 200 is configured to determine an overall user satisfaction score of a user 30 with a wellness plan based on a weighted average of each user satisfaction with each specific, granular aspect of the wellness plan. For example, as shown in FIG. 10, the overall user satisfaction score is "medium" based on a weighted average of each of the following: (1) a high user satisfaction with a medication aspect of the wellness plan, (2) a low user satisfaction with an exercise aspect of the wellness plan, (3) a high user satisfaction with a hardware/ software aspect of the wellness plan, and (4) a low user satisfaction with a human-interaction aspect of the wellness plan.

For each specific, granular aspect of the wellness plan that the user 30 is dissatisfied with, the recommendation generator 271 generates a corresponding corrective actionable recommendation 590 that addresses the user dissatisfaction with this specific, granular aspect. As shown in FIG. 10, user satisfaction of the user 30 with the exercise aspect and the human-interaction aspect is low (i.e., the user 30 is dissatisfied with these two aspects). The recommendation generator 271 may recommend that the user 30 decrease anaerobic exercise to address his/her dissatisfaction with the exercise aspect. The recommendation generator 271 may further recommend that the user 30 increase phone calls with a wellness manager to address his/her dissatisfaction with the human-interaction aspect.

In one embodiment, the satisfaction determination system 200 is configured to determine which aspect of a wellness plan is most effective based on each satisfaction level corresponding to each aspect of the wellness plan. The recommendation generator 271 may generate a recommendation that includes information indicative of the most effective aspect of the wellness plan, as determined by the satisfaction determination system 200.

In one embodiment, the satisfaction determination system 200 may adapt/change its execution in order to improve data processing for one or more specific application scenarios. For example, in medical application scenarios, it is important to quickly and accurate determine whether a user 30 recovering from heart surgery is dissatisfied with his/her wellness plan. For critical application scenarios (e.g., medical application scenarios), the satisfaction determination system 200 may collect data more frequently and more thoroughly (i.e., collect more expansive types of data) to improve accuracy of user satisfaction determination.

In another embodiment, the satisfaction determination system 200 may generate recommendations that focus directly only on the most important aspects of a wellness plan. For example, a medical-grade application may require only recommendations for medication and exercise aspects of the wellness plan; recommendations for ancillary aspects of the wellness plan, such as hardware/software aspect, may be omitted.

In another embodiment, it is more critical for the satisfaction determination system 200 to determine user dissatisfaction than user satisfaction. The importance of user satisfaction and user dissatisfaction may not be equal across different application scenarios. For example, in a medical application scenario, determining user dissatisfaction may be more critical compared to a casual fitness application scenario; the satisfaction determination system 200 may place emphasis on software user interface and user experience to highlight user dissatisfaction, and the alert engine 280 may send an alert in response to determining user dissatisfaction. By comparison, in the casual fitness application scenario, determining user satisfaction may be more important; the satisfaction determination system 200 may place emphasis on software user interface and user experience to highlight user satisfaction, and the alert engine 280 may send an alert in response to determining user satisfaction.

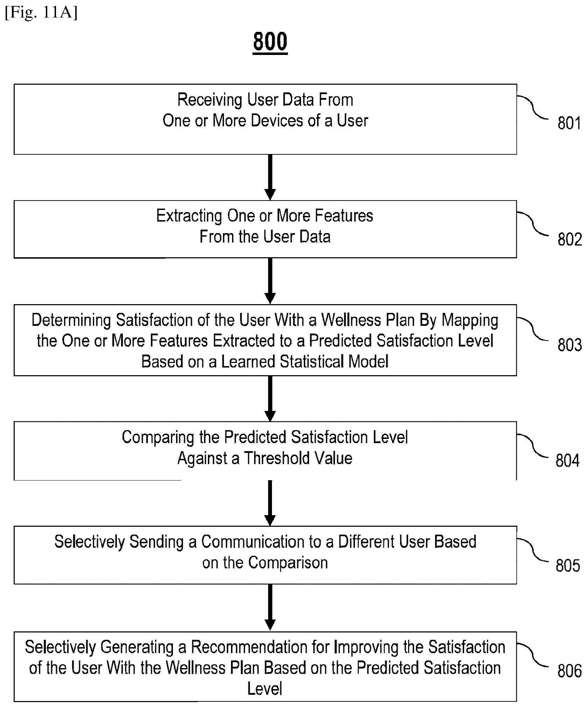

FIG. 11A is a flowchart of an example process 800 for determining user satisfaction, in one or more embodiments. In process block 801, receive user data from one or more devices of a user. In process block 802, extract one or more features from the user data.

In process block 803, determine satisfaction of the user with a wellness plan by mapping the one or more features extracted to a predicted satisfaction level based on a learned statistical model. In process block 804, compare the predicted satisfaction level against a threshold value.

In process block 805, selectively send a communication to a different user based on the comparison. In process block 806, selectively generate a recommendation for improving the satisfaction of the user with the wellness plan based on the predicted satisfaction level.

In one embodiment, process blocks 801-806 may be performed by at least the data collector 250, the satisfaction determination engine 260, the personalized corrective recommendation engine 270, and the alert engine 280.

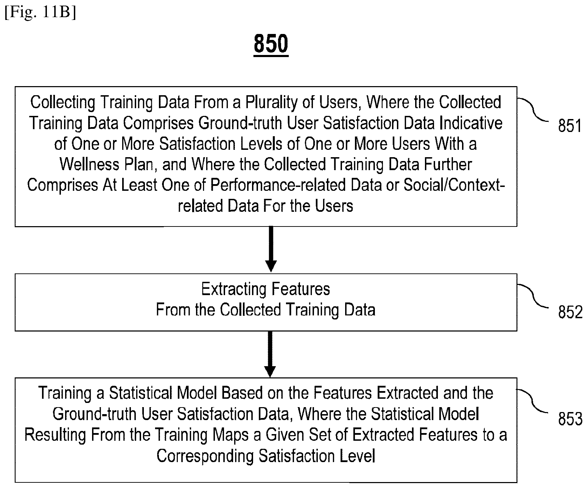

FIG. 11B is a flowchart of an example process 850 for training a statistical machine learning model for use in determining user satisfaction, in one or more embodiments. In process block 851, collect training data from a plurality of users. The collected training data comprises ground-truth user satisfaction data indicative of one or more satisfaction levels of one or more users with a wellness plan. The collected training data further comprises at least one of performance-related data or social/context-related data for the users.

In process block 852, extract features from the collected training data.

In process block 853, train a statistical model based on the features extracted and the ground-truth user satisfaction data. The statistical model resulting from the training maps each feature extracted to a corresponding satisfaction level.

In one embodiment, process blocks 851-853 may be performed by at least the training data collector 255 and the satisfaction determination training engine 265.

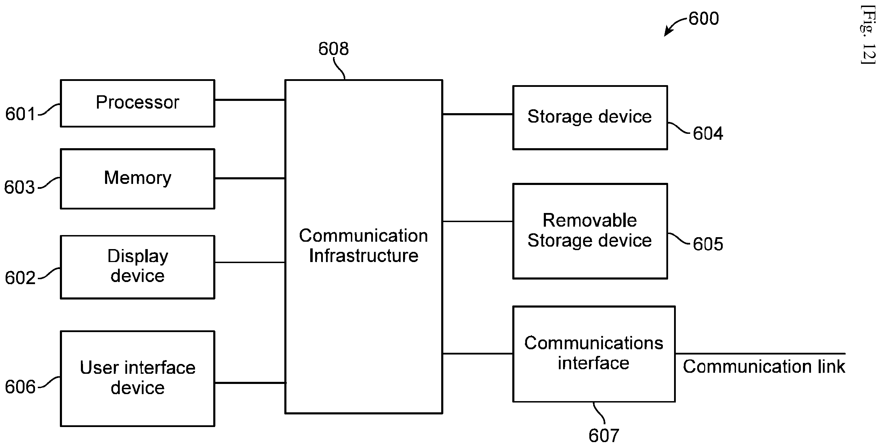

FIG. 12 is a high-level block diagram showing an information processing system comprising a computer system 600 useful for implementing the disclosed embodiments. One or more components of the satisfaction determination system 200 (e.g., the training data collector 255, the satisfaction determination training engine 265, the data collector 250, the satisfaction determination engine 260, the personalized corrective recommendation engine 270, and/or the alert engine 280) may be implemented using the computer system 600. The computer system 600 may be incorporated in a device 50 or a server device 110. The computer system 600 includes one or more processors 601, and can further include an electronic display device 602 (for displaying video, graphics, text, and other data), a main memory 603 (e.g., random access memory (RAM)), storage device 604 (e.g., hard disk drive), removable storage device 605 (e.g., removable storage drive, removable memory module, a magnetic tape drive, optical disk drive, computer readable medium having stored therein computer software and/or data), viewer interface device 606 (e.g., keyboard, touch screen, keypad, pointing device), and a communication interface 607 (e.g., modem, a network interface (such as an Ethernet card), a communications port, or a PCMCIA slot and card). The communication interface 607 allows software and data to be transferred between the computer system and external devices. The computer system 600 further includes a communications infrastructure 608 (e.g., a communications bus, cross-over bar, or network) to which the aforementioned devices/modules 601 through 607 are connected.

Information transferred via communications interface 607 may be in the form of signals such as electronic, electromagnetic, optical, or other signals capable of being received by communications interface 607, via a communication link that carries signals and may be implemented using wire or cable, fiber optics, a phone line, a cellular phone link, an radio frequency (RF) link, and/or other communication channels. Computer program instructions representing the block diagram and/or flowcharts herein may be loaded onto a computer, programmable data processing apparatus, or processing devices to cause a series of operations performed thereon to generate a computer implemented process. In one embodiment, processing instructions for process 800 (FIG. 11A) and processing instructions for process 850 (FIG. 11B) may be stored as program instructions on the memory 603, storage device 604 and the removable storage device 605 for execution by the processor 601.

Embodiments have been described with reference to flowchart illustrations and/or block diagrams of methods, apparatus (systems) and computer program products. Each block of such illustrations/diagrams, or combinations thereof, can be implemented by computer program instructions. The computer program instructions when provided to a processor produce a machine, such that the instructions, which execute via the processor create means for implementing the functions/operations specified in the flowchart and/or block diagram. Each block in the flowchart /block diagrams may represent a hardware and/or software module or logic. In alternative implementations, the functions noted in the blocks may occur out of the order noted in the figures, concurrently, etc.

The terms "computer program medium," "computer usable medium," "computer readable medium", and "computer program product," are used to generally refer to media such as main memory, secondary memory, removable storage drive, a hard disk installed in hard disk drive, and signals. These computer program products are means for providing software to the computer system. The computer readable medium allows the computer system to read data, instructions, messages or message packets, and other computer readable information from the computer readable medium. The computer readable medium, for example, may include non-volatile memory, such as a floppy disk, ROM, flash memory, disk drive memory, a CD-ROM, and other permanent storage. It is useful, for example, for transporting information, such as data and computer instructions, between computer systems. Computer program instructions may be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

As will be appreciated by one skilled in the art, aspects of the embodiments may be embodied as a system, method or computer program product. Accordingly, aspects of the embodiments may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the embodiments may take the form of a computer program product embodied in one or more computer readable medium(s) having computer readable program code embodied thereon.

Any combination of one or more computer readable medium(s) may be utilized. The computer readable medium may be a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

Computer program code for carrying out operations for aspects of one or more embodiments may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of one or more embodiments are described above with reference to flowchart illustrations and/or block diagrams of methods, apparatus (systems) and computer program products. It will be understood that each block of the flowchart illustrations and/or block diagrams, and combinations of blocks in the flowchart illustrations and/or block diagrams, can be implemented by computer program instructions. These computer program instructions may be provided to a special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

The flowchart and block diagrams in the Figures illustrate the architecture, functionality, and operation of possible implementations of systems, methods, and computer program products according to various embodiments. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

References in the claims to an element in the singular is not intended to mean "one and only" unless explicitly so stated, but rather "one or more." All structural and functional equivalents to the elements of the above-described exemplary embodiment that are currently known or later come to be known to those of ordinary skill in the art are intended to be encompassed by the present claims. No claim element herein is to be construed under the provisions of 35 U.S.C. section 112, sixth paragraph, unless the element is expressly recited using the phrase "means for" or "step for."

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The corresponding structures, materials, acts, and equivalents of all means or step plus function elements in the claims below are intended to include any structure, material, or act for performing the function in combination with other claimed elements as specifically claimed. The description of the embodiments has been presented for purposes of illustration and description, but is not intended to be exhaustive or limited to the embodiments in the form disclosed. Many modifications and variations will be apparent to those of ordinary skill in the art without departing from the scope and spirit of the invention.

Though the embodiments have been described with reference to certain versions thereof; however, other versions are possible. Therefore, the scope of the appended claims should not be limited to the description of the preferred versions contained herein.

## Claims

1. A method comprising:
Receiving, by a processor of a server (110), user data from one or more devices of a user (30) comprising at least one of:
i) a medical health record;
ii) measurable physical activity data;
iii) social networking and/or online usage data; and
iv) contextual cues data,
determining, by the processor of the server (110), a satisfaction level of the user (30) with a wellness plan (420) based on a learned statistical model (530) which is a trained machine learning model; and
selectively sending, by the processor of the server (110), a communication to a different user based on the satisfaction level,
wherein the determining a satisfaction level of the user (30) with a wellness plan (420) based on a learned statistical model (530) comprises:
extracting one or more features from the user data; and
mapping the one or more features extracted to a predicted satisfaction level based on the learned statistical model (530) which is trained based on extracted features of training data corresponding to the one or more features extracted from the user data,
wherein the learned statistical model (530) is trained based on extracted features from the training data comprising at least one of performance-related data and social/context-related data for the plurality of users and a ground-truth user satisfaction data indicative of one or more satisfaction levels of the plurality of users with the wellness plan.

2. The method of claim 1, further comprising:
determining a recommendation for improving the satisfaction level of the user (30).

3. The method of claim 1, further comprising:
determining, on a device of the user (30), a recommendation for improving the satisfaction level of the user based on one or more requirements of the wellness plan (420) and data captured by the device, wherein the data captured includes sensitive user data, and the recommendation is provided to the user (30) for review.

4. The method of claim 1, wherein the satisfaction level is indicative of a likelihood the user (30) will complete the wellness plan.

5. The method of claim 1, wherein selectively sending a communication to a different user based on the satisfaction level of the user comprises:
comparing the satisfaction level of the user (30) against a threshold value; and
selectively sending an alert to the different user based on the comparison.

6. The method of claim 1, wherein the different user is one of a wellness manager, a family member, or a friend.

7. A system, comprising:
at least one processor; and
a non-transitory processor-readable memory device storing instructions
that when executed by the at least one processor causes the at least one processor to perform operations including:
receiving user data from one or more devices of a user (30) comprising at least one of:
i) a medical health record;
ii) measurable physical activity data;
iii) social networking and/or online usage data; and
iv) contextual cues data;
determining a satisfaction level of the user (30) with a wellness plan (420) based on a learned statistical model (530) which is a trained machine learning model; and
selectively sending a communication to a different user based on the satisfaction level,
wherein the determining a satisfaction level of the user (30) with a wellness plan (420) based on a learned statistical model (530) comprises:
extracting one or more features from the user data; and
mapping the one or more features extracted to a predicted satisfaction level based on the learned statistical model (530) which is trained based on extracted features of training data corresponding to the one or more features extracted from the user data,
wherein the learned statistical model (530) is trained based on extracted features from the training data comprising at least one of performance-related data and social/context-related data for the plurality of users and a ground-truth user satisfaction data indicative of one or more satisfaction levels of the plurality of users with the wellness plan.

8. The system of claim 7, the operations further comprising:
determining a recommendation for improving the satisfaction level of the user (30).

9. The system of claim 7, wherein the satisfaction level is indicative of a likelihood the user (30) will complete the wellness plan.

10. The system of claim 7, wherein selectively sending a communication to a different user based on the satisfaction level of the user (30) comprises:
comparing the satisfaction level of the user (30) against a threshold value; and
selectively sending an alert to the different user based on the comparison.

11. The system of claim 7, wherein the different user is one of a wellness manager, a family member, or a friend.

12. The system of claim 7, wherein determining a satisfaction level of the user (30) with a wellness plan based on a learned statistical model (530) comprises:
for each aspect of the wellness plan, determining a corresponding satisfaction level of the user (30) in relation to the aspect; and
determining an overall satisfaction level of the user (30) with the wellness plan based on a weighted average of each satisfaction level corresponding to each aspect of the wellness plan.

## Patentansprüche

1. Verfahren, umfassend:
Empfangen von Benutzerdaten, die mindestens eines von Folgendem umfassen, durch einen Prozessor eines Servers (110) von einer oder mehreren Vorrichtungen eines Benutzers (30):
i) eine Krankenakte;
ii) messbare Daten zur körperlichen Aktivität;
iii) Daten zu sozialen Netzwerken und/oder zur Online-Nutzung; und
iv) kontextuelle Hinweisdaten,
Bestimmen eines Zufriedenheitsgrads des Benutzers (30) mit einem Wellnessplan (420) auf Grundlage eines erlernten statistischen Modells (530), bei dem es sich um ein trainiertes Modell des maschinellen Lernens handelt, durch den Prozessor des Servers (110); und
selektives Senden einer Kommunikation auf Grundlage des Zufriedenheitsgrads durch den Prozessor des Servers (110) an einen anderen Benutzer,
wobei das Bestimmen eines Zufriedenheitsgrads des Benutzers (30) mit einem Wellnessplan (420) auf Grundlage eines erlernten statistischen Modells (530) Folgendes umfasst:
Extrahieren eines oder mehrerer Merkmale aus den Benutzerdaten; und
Zuordnen der einen oder mehreren extrahierten Merkmale zu einem vorhergesagten Zufriedenheitsgrad auf Grundlage des erlernten statistischen Modells (530), das auf Grundlage von extrahierten Merkmalen von Trainingsdaten trainiert ist, die den einen oder mehreren aus den Benutzerdaten extrahierten Merkmalen entsprechen,
wobei das erlernte statistische Modell (530) auf Grundlage von extrahierten Merkmalen aus den Trainingsdaten trainiert wird, die mindestens eines von leistungsbezogenen Daten und sozialen/kontextbezogenen Daten für die Vielzahl von Benutzern und Ground-Truth-Benutzerzufriedenheitsdaten umfassen, die auf einen oder mehrere Zufriedenheitsgrade der Vielzahl von Benutzern mit dem Wellnessplan hinweisen.

2. Verfahren nach Anspruch 1, ferner umfassend:
Bestimmen einer Empfehlung zum Verbessern des Zufriedenheitsgrads des Benutzers (30).

3. Verfahren nach Anspruch 1, ferner umfassend:
Bestimmen einer Empfehlung zum Verbessern des Zufriedenheitsgrads des Benutzers auf einer Vorrichtung des Benutzers (30) auf Grundlage von einer oder mehreren Anforderungen des Wellnessplans (420) und durch die Vorrichtung erfassten Daten, wobei die erfassten Daten vertrauliche Benutzerdaten beinhalten und die Empfehlung dem Benutzer (30) zur Überprüfung bereitgestellt wird.

4. Verfahren nach Anspruch 1, wobei der Zufriedenheitsgrad ein Hinweis auf eine Wahrscheinlichkeit ist, dass der Benutzer (30) den Wellnessplan abschließen wird.

5. Verfahren nach Anspruch 1, wobei das selektive Senden einer Kommunikation an einen anderen Benutzer auf Grundlage des Zufriedenheitsgrads des Benutzers Folgendes umfasst:
Vergleichen des Zufriedenheitsgrads des Benutzers (30) mit einem Schwellenwert; und
Selektives Senden einer Warnung an den anderen Benutzer auf Grundlage des Vergleichs.

6. Verfahren nach Anspruch 1, wobei der andere Benutzer einer von einem Wellness-Manager, einem Familienmitglied oder einem Freund ist.

7. System, umfassend:
mindestens einen Prozessor; und
eine nicht transitorische prozessorlesbare Speichervorrichtung, die Anweisungen speichert, die bei Ausführung durch den mindestens einen Prozessor den mindestens einen Prozessor dazu veranlassen, Vorgänge durchzuführen, die Folgendes beinhalten:
Empfangen von Benutzerdaten, die mindestens eines von Folgendem umfassen, von einem oder mehreren Vorrichtungen eines Benutzers (30):
i) eine Krankenakte;
ii) messbare Daten zur körperlichen Aktivität;
iii) Daten zu sozialen Netzwerken und/oder zur Online-Nutzung; und
iv) kontextuelle Hinweisdaten;
Bestimmen eines Zufriedenheitsgrads des Benutzers (30) mit einem Wellnessplan (420) auf Grundlage eines erlernten statistischen Modells (530), bei dem es sich um ein trainiertes Modell des maschinellen Lernens handelt; und
selektives Senden einer Kommunikation auf Grundlage des Zufriedenheitsgrads an einen anderen Benutzer,
wobei das Bestimmen eines Zufriedenheitsgrads des Benutzers (30) mit einem Wellnessplan (420) auf Grundlage eines erlernten statistischen Modells (530) Folgendes umfasst:
Extrahieren eines oder mehrerer Merkmale aus den Benutzerdaten; und
Zuordnen der einen oder mehreren extrahierten Merkmale zu einem vorhergesagten Zufriedenheitsgrad auf Grundlage des erlernten statistischen Modells (530), das auf Grundlage von extrahierten Merkmalen von Trainingsdaten trainiert ist, die den einen oder mehreren aus den Benutzerdaten extrahierten Merkmalen entsprechen,
wobei das erlernte statistische Modell (530) auf Grundlage von extrahierten Merkmalen aus den Trainingsdaten trainiert wird, die mindestens eines von leistungsbezogenen Daten und sozialen/kontextbezogenen Daten für die Vielzahl von Benutzern und Ground-Truth-Benutzerzufriedenheitsdaten umfassen, die auf einen oder mehrere Zufriedenheitsgrade der Vielzahl von Benutzern mit dem Wellnessplan hinweisen.

8. System nach Anspruch 7, wobei die Vorgänge ferner Folgendes umfassen:
Bestimmen einer Empfehlung zum Verbessern des Zufriedenheitsgrads des Benutzers (30).

9. System nach Anspruch 7, wobei der Zufriedenheitsgrad ein Hinweis auf eine Wahrscheinlichkeit ist, dass der Benutzer (30) den Wellnessplan abschließen wird.

10. System nach Anspruch 7, wobei das selektive Senden einer Kommunikation an einen anderen Benutzer auf Grundlage des Zufriedenheitsgrads des Benutzers (30) Folgendes umfasst:
Vergleichen des Zufriedenheitsgrads des Benutzers (30) mit einem Schwellenwert; und
Selektives Senden einer Warnung an den anderen Benutzer auf Grundlage des Vergleichs.

11. System nach Anspruch 7, wobei der andere Benutzer einer von einem Wellness-Manager, einem Familienmitglied oder einem Freund ist.

12. System nach Anspruch 7, wobei das Bestimmen eines Zufriedenheitsgrads des Benutzers (30) mit einem Wellnessplan auf Grundlage eines erlernten statistischen Modells (530) Folgendes umfasst:
für jeden Aspekt des Wellnessplans, Bestimmen eines entsprechenden Zufriedenheitsgrads des Benutzers (30) in Bezug auf den Aspekt; und
Bestimmen eines allgemeinen Zufriedenheitsgrads des Benutzers (30) mit dem Wellnessplan auf Grundlage eines gewichteten Durchschnitts jedes Zufriedenheitsgrads, der jedem Aspekt des Wellnessplans entspricht.

## Revendications

1. Procédé comprenant :
la réception, par un processeur d'un serveur (110), de données utilisateur en provenance d'un ou plusieurs dispositifs d'un utilisateur (30) comprenant au moins un parmi :
i) un dossier médical ;
ii) des données d'activité physique mesurables ;
iii) des données de réseautage social et/ou d'utilisation en ligne ; et
iv) des données d'indices contextuels,
la détermination, par le processeur du serveur (110), d'un niveau de satisfaction de l'utilisateur (30) avec un plan de bien-être (420) basé sur un modèle statistique appris (530) qui est un modèle d'apprentissage automatique formé ; et
l'envoi sélectif, par le processeur du serveur (110), d'une communication à un utilisateur différent sur la base du niveau de satisfaction,
ladite détermination d'un niveau de satisfaction de l'utilisateur (30) avec un plan de bien-être (420) basé sur
un modèle statistique appris (530) comprenant :
l'extraction d'une ou plusieurs caractéristiques des données d'utilisateur ; et
la mise en correspondance de la ou des caractéristiques extraites avec un niveau de satisfaction prédit sur la base du modèle statistique appris (530) qui est formé sur la base de caractéristiques extraites de données de formation correspondant à la ou aux caractéristiques extraites des données d'utilisateur,
ledit modèle statistique appris (530) étant formé sur la base de caractéristiques extraites des données de formation comprenant au moins l'une des données liées à la performance et des données sociales/liées au contexte pour la pluralité d'utilisateurs et des données de satisfaction d'utilisateur de réalité de terrain indiquant un ou plusieurs niveaux de satisfaction de la pluralité d'utilisateurs avec le plan de bien-être.

2. Procédé de la revendication 1, comprenant en outre :
la détermination d'une recommandation destinée à améliorer le niveau de satisfaction de l'utilisateur (30).

3. Procédé de la revendication 1, comprenant en outre :
la détermination, sur un dispositif de l'utilisateur (30), d'une recommandation destinée à améliorer le niveau de satisfaction de l'utilisateur sur la base d'une ou plusieurs exigences du plan de bien-être (420) et des données capturées par le dispositif, lesdites données capturées comprenant des données utilisateur sensibles, et ladite recommandation étant fournie à l'utilisateur (30) pour examen.

4. Procédé de la revendication 1, ledit niveau de satisfaction indiquant une probabilité que l'utilisateur (30) achève le plan de bien-être.

5. Procédé de la revendication 1, ledit envoi sélectif d'une communication à un utilisateur différent sur la base du niveau de satisfaction de l'utilisateur comprenant :
la comparaison du niveau de satisfaction de l'utilisateur (30) avec une valeur seuil ; et
l'envoi sélectif d'une alerte à l'utilisateur différent sur la base de la comparaison.

6. Procédé de la revendication 1, ledit utilisateur différent étant l'un d'un gestionnaire de bien-être, d'un membre de la famille ou d'un ami.

7. Système, comprenant :
au moins un processeur ; et
un dispositif de mémoire lisible par processeur non transitoire stockant des instructions qui, lorsqu'elles sont exécutées par l'au moins un processeur, amènent l'au moins un processeur à réaliser des opérations comprenant :
la réception de données utilisateur en provenance d'un ou plusieurs dispositifs d'un utilisateur (30) comprenant au moins un parmi :
i) un dossier médical ;
ii) des données d'activité physique mesurables ;
iii) des données de réseautage social et/ou d'utilisation en ligne ; et
iv) des données d'indices contextuels ;
la détermination d'un niveau de satisfaction de l'utilisateur (30) avec un plan de bien-être (420) basé sur un modèle statistique appris (530) qui est un modèle d'apprentissage automatique formé ; et
l'envoi sélectif d'une communication à un utilisateur différent sur la base du niveau de satisfaction, ladite détermination d'un niveau de satisfaction de l'utilisateur (30) avec un plan de bien-être (420) basé sur
un modèle statistique appris (530) comprenant :
l'extraction d'une ou plusieurs caractéristiques des données d'utilisateur ; et
la mise en correspondance de la ou des caractéristiques extraites avec un niveau de satisfaction prédit sur la base du modèle statistique appris (530) qui est formé sur la base de caractéristiques extraites de données de formation correspondant à la ou aux caractéristiques extraites des données d'utilisateur,
ledit modèle statistique appris (530) étant formé sur la base de caractéristiques extraites des données de formation comprenant au moins l'une des données liées à la performance et des données sociales/liées au contexte pour la pluralité d'utilisateurs et des données de satisfaction d'utilisateur de réalité de terrain indiquant un ou plusieurs niveaux de satisfaction de la pluralité d'utilisateurs avec le plan de bien-être.

8. Système de la revendication 7, lesdites opérations comprenant en outre :
la détermination d'une recommandation destinée à améliorer le niveau de satisfaction de l'utilisateur (30).

9. Système de la revendication 7, ledit niveau de satisfaction indiquant une probabilité que l'utilisateur (30) achève le plan de bien-être.

10. Système de la revendication 7, ledit envoi sélectif d'une communication à un utilisateur différent sur la base
du niveau de satisfaction de l'utilisateur (30) comprenant :
la comparaison du niveau de satisfaction de l'utilisateur (30) avec une valeur seuil ; et
l'envoi sélectif d'une alerte à l'utilisateur différent sur la base de la comparaison.

11. Système de la revendication 7, ledit utilisateur différent étant l'un d'un gestionnaire de bien-être, d'un membre de la famille
ou d'un ami.

12. Système de la revendication 7, ladite détermination d'un niveau de satisfaction de l'utilisateur (30) avec un plan de bien-être basé sur un modèle statistique appris (530) comprenant :
pour chaque aspect du plan de bien-être, la détermination d'un niveau de satisfaction correspondant de l'utilisateur (30) par rapport à l'aspect ; et
la détermination d'un niveau de satisfaction global de l'utilisateur (30) avec le plan de bien-être sur la base d'une moyenne pondérée de chaque niveau de satisfaction correspondant à chaque aspect du plan de bien-être.
